# EUROPEAN PATENT APPLICATION

(11) **EP 1 780 218 A1**
(43) Date of publication of application: **02.05.2007**
(21) Application number: 05761905.8
(22) Date of filing: 21.06.2005
(51) Int. Cl.: C07K 14/475

(54) **USE OF AMPHIREGULIN AS A PROTECTIVE AGENT IN ACUTE HEPATIC INJURY**

(30) Priority: 20.07.2004 ES 200401776
(71) Applicant: Proyecto de Biomedicina Cima, S.L., 31003 Pamplona (Navarra) (ES)
(72) Inventor: AVILA ZARAGOZA, Matias Antonio, E-31008 Pamplona (ES); RUIZ GARCÍA-TREVIJANO, Elena, E-31008 Pamplona (ES); BERASAIN LASARTE, Carmen, E-31008 Pamplona (ES); PRIETO VALTUEÑA, Jesus, E-31008 Pamplona (ES)
(74) Representative: Ungria Lopez, Javier
(86) International application number: PCT/ES2005/000348
(87) International publication number: WO 2006/021599

(57) **Abstract**

The invention relates to the use of amphiregulin in the production of a medicament which can be used to treat acute hepatic injury and which is administered, for example, in order to: promote a primary endogenous protective reaction in the hepatic tissue against acute hepatic injury, promote DNA synthesis in hepatocytes, prevent the death of hepatocytes in the hepatic tissue of patients with acute hepatic injury, stimulate the regeneration of the remaining hepatic parenchyma following an acute hepatic injury of any aetiology, and stimulate hepatic regeneration following a partial hepatectomy. According to the invention, the amphiregulin is administered as a hepatoprotector medicine for patients with acute hepatic injury of any aetiology and/or as a hepatoprotector medicine and stimulant of hepatocytic regeneration for recipients of a liver transplant from a living donor or a cadaver donor.

## Description

### TECHNICAL FIELD OF THE INVENTION

The present invention belongs to the field of biotechnology applied to the medical-pharmaceutical sector for the treatment of liver diseases, and particularly of acute liver damage such as for example acute liver failure (ALF).

More specifically, the present invention provides a new treatment for such disease based on the use of amphiregulin (AR).

### STATE OF THE ART PRIOR TO THE INVENTION

Acute liver damage is an extremely serious disorder that may be expressed as acute liver failure (ALF) secondary to the loss of functional liver mass due to hepatocyte death. ALF is not a disease as such but rather a syndrome with a severity proportional to the degree of hepatocyte loss. The disorder is dramatic, and has complications with multiorganic repercussions that include encephalopathy, brain edema, sepsis, respiratory and renal failure, intestinal bleeding and cardiovascular collapse [Sanyal, A.J., Stravitz, R.T. The liver. Chapter 16. Pages: 445-496. Zakim and Boyer Eds. Saunders. Philadelphia. 2003].

While ALF is not particularly common, the associated mortality rate is between 40 and 95% [Sanyal, A.J., Stravitz, R.T. The liver. Chapter 16. Pages: 445-496. Zakim and Boyer Eds. Saunders. Philadelphia. 2003; and Galun, E., Axelrod, J.H. Biochim. Biophys. Acta. 1592:345-358.2002 (identified as (15) below)].

The etiology of ALF is diverse, with geographical variability; its correct definition is important in order to establish a prognosis and apply treatment.

Among the agents that may cause ALF, mention should be made of hepatitis viruses, certain drugs and toxins, metabolic disorders, some cases of acute ischemia, and massive resection of hepatic parenchyma [Sanyal, A.J. ibid; Galun, E. *ibid*].

The successful resolution of ALF depends on the possibility of inhibiting hepatocellular damage and on the regeneration of damaged parenchyma. Most therapeutic resources available in current clinical practice attempt to palliate the multiorganic manifestations of ALF; however, no therapeutic strategies are able to reduce necrosis and apoptosis, or promote hepatocyte regeneration - liver transplantation ultimately being the only possible alternative [Sanyal, A.J. ibid; Galun, E. ibid] for healing the patient.

Cytoprotective and regenerative mechanisms are known to be activated in the liver after hepatic tissue loss secondary to partial hepatectomy (PH) or damage of toxic, viral, ischemic or immune origin [(1) Michalopoulos, G.K., DeFrances, M.C. 1997. Liver regeneration. Science 276: 60-66. (2) Fausto, N. 2000. Liver regeneration. J. Hepatol. 32 (suppl 1): 19-31. (3) Taub, R.A. 2003. Hepatic regeneration. In: The Liver. D. Zakim, J.L. Boyer, Saunders, Philadelphia. U.S.A. 31-48]. Different experimental approaches have helped define the underlying mechanisms that contribute to preserve liver function and restore functional liver mass after severe hepatic damage [(4) Koniaris, L.G., McKillop, I.H., Schwartz, S.I., Zimmers, T.A. 2003. Liver regeneration. J. Am. Coll. Surg. 197: 634-659]. This complex response is mediated by a network of cytokines, comitogens and growth factors, in the context of a process that develops in a series of coordinated steps [(2), (3), (5) Kosai, K., Matsumoto, K., Nagata, S., Tsujimoto, Y., Nakamura, T. 1998. Abrogation of Fas-induced fulminant hepatic failure in mice by hepatocyte growth factor. Biochem. Biophys. Res. Commun. 244: 683-690. (6). . Éthier, C., Raymond, V-A., Musallam, L., Houle, R., and Bilodeau, M. 2003Antiapoptotic effect of EGF on mouse hepatocytes associated with downregulation of proapoptotic Bid protein. Am. J. Physiol. Gastrointest. Liver Physiol. 285: G298-G308. (7). Kanda, D., Takagi, H., Toyoda, M., Horiguchi, N., Nakajima, H., Otsuka, T., Mori, M. 2002. Transforming growth factor α protects against Fas-mediated liver apoptosis in mice. FEBS Lett. 519: 11-15]. It is considered that many of the cytokines and growth factors which are critical to regenerative response to damage or resection in animal models are also expressed in humans in the course of liver regeneration - thus suggesting preservation of the fundamental mechanisms among species (4).

At experimental level it has been shown that the administration to animals (rat and mouse) of certain growth factors and cytokines, protect against ALF, avoiding cell death and stimulating regeneration of liver parenchyma. Such factors include hepatocyte growth factor (HGF), transforming growth factor α (TGF-α) and epidermal growth factor (EGF). [Kosai, K., Matsumoto, K. Nagata, S., Tsujimoto, Y., Nalamura, T. Biochem. Biohys. Res. Commun. 244:683-690.1998 identified as (5) below; Kand, D., Takagi, H., Toyoda, M., Horiguchi, N., Nakajima, H., Otsuka, T., Mori, M. FEBS Lett. 519-11-15.2002; and Ethier, C., Raymond, V.A., Musallam, L., Houle, R., Bilodeau, M. Am. J. Physiol. 285:G298-G308.2003]. Among cytokines, mention may be made of interleukin 6 (IL-6) and cardiotrophin-1 (CT-1). [Kovalovich, K., DeAngelis, R.A., Li, W., Durth, E.E, Ciliberto, G., Taub, R. Hepatology 31:149-159.2000 identified as (26) below; and Bustos, M., Beraza, N., Lasarte, J.J., Baixeras, E., Alzuguren, P., Bordet, T., Prieto, J. Gastroenterology 125:192-201.2003 identified as (43) below]. Liver regeneration is a unique response that aims to restore liver mass following parenchymal resection or damage. The survival and proliferation signals are transmitted through a complex network of cytokines and growth factors that operate in a coordinated manner. However, despite intensive research in the last few decades, the molecules and mechanisms involved in the physiological adaptive response to liver damage have not been fully clarified.

The inventors have recently observed that Wilms' tumor suppressor gene *WT1* is induced in the liver of patients with hepatocellular damage, as well as in the liver of rats treated with carbon tetrachloride (CCl₄) [(8) Berasain, C., Herrero, J.I., Garcia-Trevijano, E.R., Avila, M.A., Esteban, J.I., Mato, J.M., and Prieto, J. 2003. Expression of Wilms' tumor suppressor in the cirrhotic liver: relationship to HNF4 levels and hepatocellular function. Hepatology 38: 148-157]. The *WT1* gene encodes for a transcription factor possessing zinc fingers that can regulate the expression of a range of genes related to growth and differentiation [(9) Scharnhorst, V., Van der Eb, A.J, and Jochemsen, A.G. WT1 proteins: functions in growth and differentiation. 2001]. Gene 273:141-161].

One of the main physiological targets directly induced by *WT1* is amphiregulin (AR) [(10) Lee, S.B., Huang, K., Palmer, R., Truong, V.B., Herzlinger, D., Kolquist, K.A., Wong, J., Paulding, C., Yoon, S.K., Gerald, W., Oliner, J.D., and Haber, D.A. 1999. The Wilms' tumor suppressor WT1 encodes a transcriptional activator of amphiregulin. Cell 98: 663-673]. AR is a polypeptide growth factor belonging to the EGF family, and a ligand of EGF receptor (EGF-R), that was originally isolated from conditioned media from MCF-7 human breast carcinoma cells treated with phorbol 12-myristate 13-acetate [(11) Shoyab, M., McDonald, V.L., Bradley, G., and Todaro, G.J. 1988. Amphiregulin: a bifunctional growth-modulating glycoprotein produced by the phorbol 12-myristate 13-acetate-treated human breast adenocarcinoma cell line MCF-7. Proc. Natl. Acad. Sci. USA. 85: 6528-6532]. In the same way as EGF and TGFα, AR is synthesized as a transmembrane precursor that is proteolytically processed to yield the mature secreted form [(12) Lee, D.C., Sunnarborg, S.W., Hinkle, C.L., Myers, T.J., Stevenson, M.Y., Russell, W.E, Castner, B.J., Gerhart, M.J., Paxton, R.J., Black, R.A., Chang, A., and Jackson, L.F. 2003. TACE/ADAM17 processing of EGF-R ligands indicates a role as a physiological convertase. Ann. N.Y. Acad. Sci. 995: 22-38]. Expression of AR is tissue-specific. In humans it has been seen to be more predominant in the ovary and placenta, and is undetectable in the liver [(13) Plowman, G.D., Green, J.M., McDonald, V.L., Neubauer, M.G., Disteche, C.M., Todaro, G.J., and Shoyab, M. 1990. Amphiregulin gene encodes a novel epidermal growth factor-related protein with tumor-inhibitory activity. Mol. Cell Biol. 10: 1969-1981].

AR possesses bifunctional properties, stimulating the proliferation of a variety of normal cells and inhibiting many tumor cell lines [(10), (13) and (14) Kato, M., Inazu, T., Kawai, Y., Masamura, K., Yoshida, M., Tanaka, N., Miyamoto, K., and Miyamori, I. Amphiregulin is a potent mitogen for the vascular smooth muscle cell line, A75. 2003. Biochem. Biophys. Res. Commun. 301: 1109-1115].

US-5115096 patent describes the physico-chemical characteristics of amphiregulin and its antiproliferative effect on cancer cells of epithelial origin, as well as its use in wound treatment and in the diagnosis and treatment of cancer. Reference is made to a certain reduced level of amphiregulin production in the liver - from which it is deduced that it apparently "plays some functional role".

Patent US-5980885 describes a method involving the use of AR, together with fibroblast growth factor, to induce proliferation of precursor cells in mammalian neuronal tissues.

Patent US-6204359 describes the use of a new form of AR produced by keratinocytes in the treatment of wounds and cancer.

Patent application US-20011051358 describes the obtainment and use of polypeptides from EEGF (Extracellular/Epidermal Growth Factor) for, among other applications, the treatment of liver disorders. It also refers to the possibility of treatments in relation to liver regeneration. However, AR is only mentioned as a known product in the state of the art, which is supposedly surpassed by the invention described in this document.

Patent application WO-0145697 describes a regulating agent that inhibits AR expression, and its use for human skin treatment.

Finally, patent application WO-02102319 describes polynucleotides that encode for BGS-8 polypeptides, fragments and homologues of the latter that are of use, among other applications, for the treatment and prevention of liver disorders and proliferative states that affect the liver. It is also indicated that because of "the strong homology with EGF protein family members such as bFGF, PDGF, AR, beta-cellulin, crypto- and TGF-alfa", it is to be expected that BGS-8 polypeptide shares at least some biological activity with proteins belonging to that family.

The state of the art proves the need for new alternatives for ALF treatment. It was therefore, desirable to find a more effective treatment for ALF based on the application of a growth factor or cytokine, the administration of which could afford liver protection in ALF.

### DESCRIPTION OF THE INVENTION

Based on the state of the art commented above, the inventors have found that unexpectedly, AR administered externally is useful as a protective agent in acute liver damage which - for example - may lead to (or already have caused) acute liver failure (ALF).

Thus, an object of the present invention is the use of amphiregulin for the preparation of a medicine for the treatment of acute liver damage.

Another object of the present invention is the use of this medicine in which said factor is administerd, to reinforce a primary endogenous protective reaction of liver tissue against acute liver damage.

Yet another object of the present invention is the use of amphiregulin in the preparation of a medicine administered to promote DNA synthesis in liver parenchymal cells in acute liver damage.

Another object of the present invention is the use of AR in the manufacture of a medicine administered to prevent the death of liver parenchymal cells in acute liver damage.

Yet another object is the use of AR in the manufacture of a medicine to stimulate regeneration of remaining liver parenchyma following acute liver damage of any etiology.

Another objective of the invention is the use of AR in the manufacture of a medicine useful for stimulating liver regeneration after a partial hepatectomy.

Yet another objective of the invention is the use of AR in the manufacture of a medicine useful as a hepatoprotective drug and as a stimulator of hepatocyte regeneration in patients receptors of a liver transplanted *in vivo* or from a cadaver.

Amphiregulin is therefore useful for the treatment of acute liver damage via administration to a patient requiring such treatment. Thus, amphiregulin can be used in a method for the treatment of acute liver damage that comprises the administration of an effective amount of AR to a patient requiring such therapy. In the context of such use, the drug can be used (for example) in a treatment method where AR is administered to a patient to achieve:
* enhancement of a primary endogenous protective reaction of the liver tissue against acute liver damage; and/or
* promotion of DNA synthesis in hepatocytes during acute liver damage; and/or
* prevention of hepatocyte death during acute liver damage; and/or
* stimulation of remaing liver parenchyma regeneration after acute liver damage; and/or
* enchancement of a primary endogenous protective reaction in liver tissue against liver damage, and/or promotion of hepatocyte DNA synthesis; and/or
* prevention of hepatocyte death in liver tissue of patients with acute liver damage; and or
* stimulation of regeneration of the remaining liver parenchyma after acute liver damage of any etiology; and/or
* stimulation of liver regeneration following a partial hepatectomy; and/or
* stimulation of hepatocyte regeneration in patients receptors of a liver transplant *de vivo* or from a cadaver.

The inventors have found that, surprisingly, the administration of AR is able to induce hepatocyte survival during liver damage. Thus, it has been proven that AR behaves as a mitogenic or proliferative factor. The inventors have demonstrated that AR is able to act directly on isolated hepatocytes, promoting their proliferation and inhibiting apoptosis. These effects seem to be mediated by activation of EGF-R and extracellularly regulated kinases 1/2 (ERK1/2), signal-3 transducer and activator of transcription (STAT-3), c-jun N-terminal kinase (JNK) and Akt. Moreover, AR induces the expression of two survival mediators, TGFα and CT-1, in isolated hepatocytes.

Likewise, the inventors have been able to demonstrate that the *in vivo* administration of AR to mice subjected to acute liver damage with antibody Jo2, which specifically activates the Fas ligand receptor, or with CCl₄, two clinically relevant models of liver damage,[(4), (15) Galun, E., and Axelrod, J.H. 2002. The role of cytokines in liver failure and regeneration: potential new molecular therapies. Biochim. Biophys. Acta. 1592: 345-358] affords significant protection of liver tissue, and inhibition of apoptosis. Thus, the findings of the inventors reveal new functions for AR in the liver that reflect its therapeutic utility for reducing hepatocellular damage in cases of severe liver damage or lesions.

The above comments reflect the hepatoprotective properties of amphiregulin and its mitogenic effect in models of hepatocellular damage of relevance to human acute liver damage.

AR can be administered as an injection via parenteral route, and preferentially via intravenous route - though it can also be administered subcutaneously or intramuscularly.

The forms for parenteral administration can be obtained conventionally by mixing AR with buffers, stabilizers, preservatives, solubilizing agents, tonic agents and/or suspension agents. In order to avoid effects upon the disulfide bonds found in the AR molecule, the formulation should not include components capable of modifying (reducing) these disulfide bonds. The compositions are sterilized using known techniques, and are packaged for administration as injections.

Potential buffers comprise organophosphate-based salts.

Examples of solubilizing agents are castor oil solidified with polyoxyethylene, polysorbate 80, nicotinamide, and macrogol.

As stabilizers, sodium sulfite or metasodium sulfite, and as preservatives sorbic acid, cresol, paracresol and others, may be used.
As a preferential form of administration, the injectable composition may be a solution, an emulsion or a sterile dispersion. Said injectable formulation is prepared by AR dissolution, emulsion or dispersion together with one or more excipients, in water for injection.

Among the excipients that may form part of the injectable preparation for subcutaneous administration, mention can be made of buffers, depending on injection in tissues, with or without buffering potential, and depending on the stability of the active substance or substances at physiological pH. Among the buffers, mention can be made of regulating solutions such as citric acid - sodium citrate, acetic acid - sodium acetate, and monosodium carbonate - disodium carbonate, among others.

Other optional excipients are sterilizing agents to avoid the presence of pyrogens and/or contaminants.

Another optional component of the pharmaceutical composition for administration via the subcutaneous route is one or more liquid carrier agents such as for example water, hydrocarbons, alcohols, polyols, ethers, vegetable oils, lanolin, and methylketone, among others.

The formulations for intravenous or intraperitoneal injection, can be designed to allow the administration, in one or several doses, of 0.5 to 1.8 mg/kg patient body weight per day, such as for example 0.85 to 1.55 mg/kg patient body weight per day, and more specifically 1 to 1.5 mg/kg patient body weight per day.

### BRIEF DESCRIPTION OF THE FIGURES

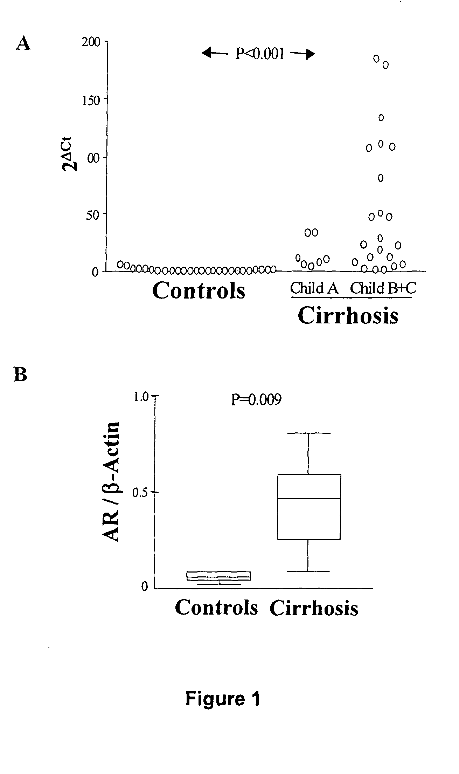

**Figure 1A:** Expression of AR gene in human and experimental liver cirrhosis, determined via real time polymerase chain reaction (RT-PCR) in liver samples from controls (n=26) and cirrhotic patients (Child-Pugh A cirrhosis, n=7, Child-Pugh B+C cirrhosis, n=22).
**Figure 1B:** Expression of AR gene in control rat liver and cirrhotic liver induced by CCl₄ as determined by RT-PCR (n=6 animals per group).

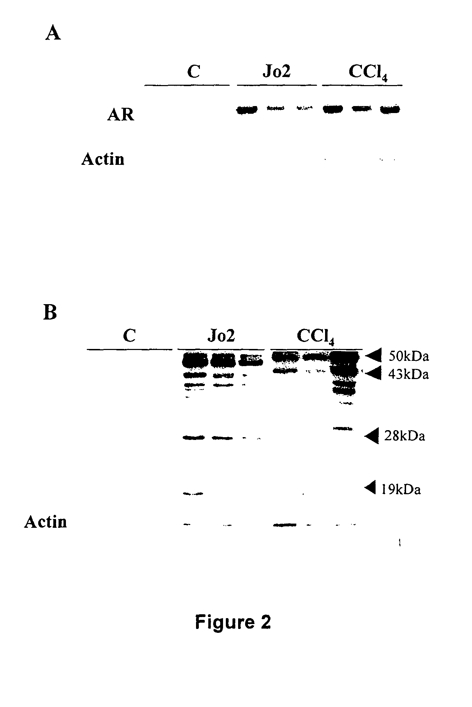

**Figure 2A:** Expression of AR in the liver of control mice and after the induction of acute liver damage by Jo2 antibodies or CCl₄. The expression of AR was evaluated by RT-PCR 5 h after administration of the mentioned treatments.
**Figure 2B:** Expression of AR in acute liver damage induced by Jo2 antibodies or CCl₄ and evaluated by Western blot. The samples of liver tissue were obtained after 12 and 24 h treatment with Jo2 and CCl₄, respectively. The analyses were carried out with affinity purified and biotinylated anti-AR antibody. The arrow tips indicate the different forms of AR, and three representative samples per group are presented. The membranes were hybridized with an antibody specific for actin as loading control. Representative images are shown.

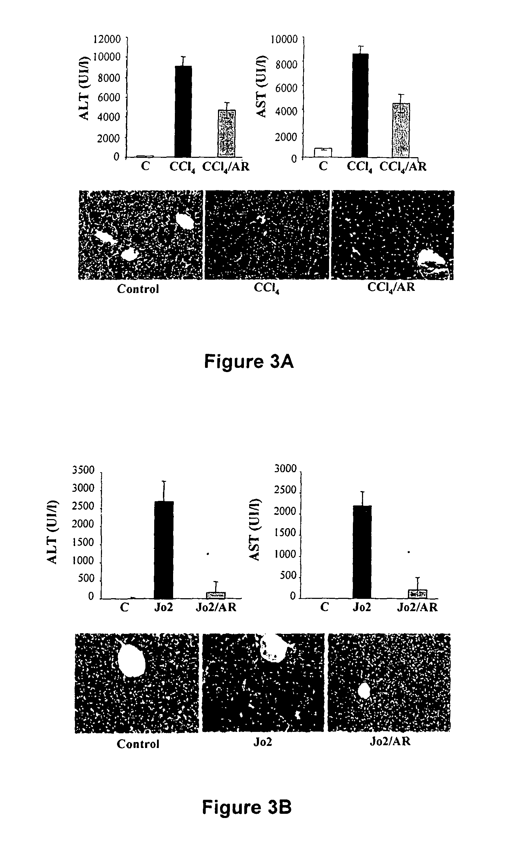

**Figure 3A:** Treatment with AR prevents acute liver damage induced by CCl₄. Liver histology is shown, along with serum transaminase levels in mice treated with CCl₄ (H&E staining, original magnification X200). Serum and liver tissue samples were collected 24 h after administration of CCl₄. Values are expressed as the mean ± SEM of three experiments performed in triplicate.
**Figure 3B:** Effect of AR treatment on liver histology and serum transaminases in mice treated with Jo2 (H&E staining, original magnification X200). The samples of serum and liver tissue were obtained 12 h after Jo2 administration. Values are expressed as the mean ± SEM of three experiments performed in triplicate. The asterisk indicates *P*<0.01 *vs* Jo2 alone.

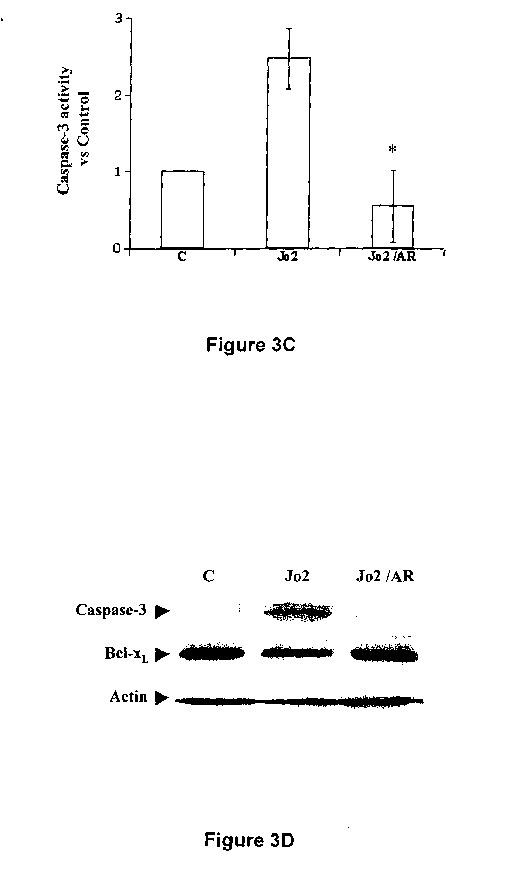

**Figure 3C:** Effect of AR treatment on caspase-3 activity in mouse liver measured 12 h after Jo2 injection. Values are expressed as the mean ± SEM of three experiments performed in triplicate, and the asterisk indicates P<0.01 *vs* Jo2 alone.
**Figure 3D**: Effect of AR treatment on the levels of p17 subunit of active caspase-3 and of Bcl-x_{L} protein in liver of control mice -C- and in extracts of liver obtained 12 h after Jo2 injection. The membranes were hybridized with an antibody specific for actin as loading control. Representative images are shown.

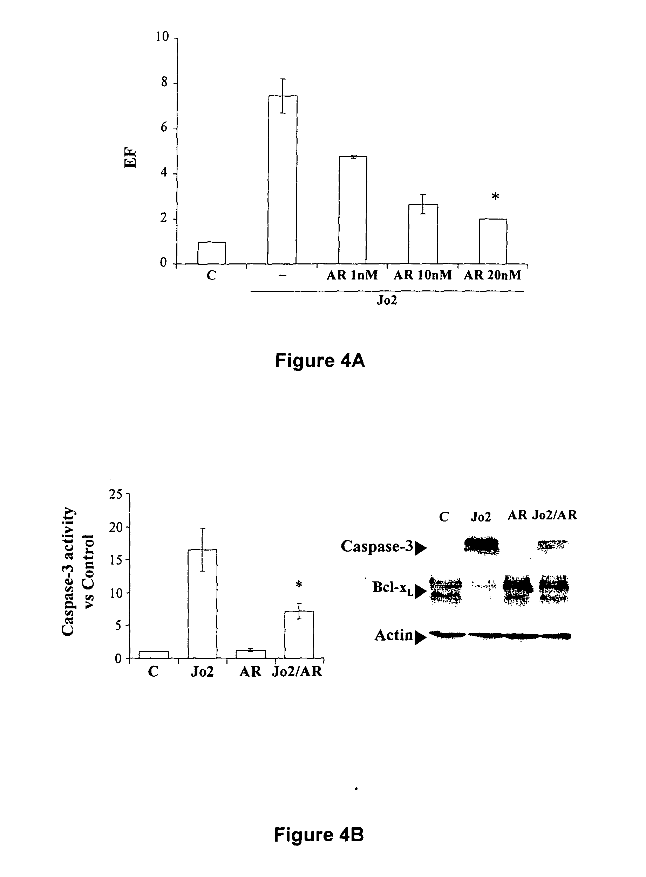

**Figure 4A:** Antiapoptotic effect of AR on mouse hepatocytes in primary culture. Apoptosis was induced by treatment with actinomycin D and Jo2 in the presence of increasing concentrations of AR. Apoptosis was evaluated by measuring specific enrichment in mono- and oligonucleosomes release in the cytoplasm (enrichment factor: EF). Values normalized with respect to those obtained in control hepatocytes -C-. Values are expressed as the mean ±SEM of three experiments performed in triplicate, and the asterisk indicates P<0.05 *vs* Jo2.
**Figure 4B:** Left panel: Effect of AR (20 nM) on caspase-3 activity in cultured mouse hepatocytes treated with actinomycin D and Jo2. Values are expressed as the mean ± SEM of three experiments performed in triplicate, and the asterisk indicates P<0.05 vs cells treated with actinomycin D and Jo2. Right panel: Western blot analysis of the active p17 subunit of caspase-3 and of Bcl-x_{L} protein in the same samples described in the left panel.

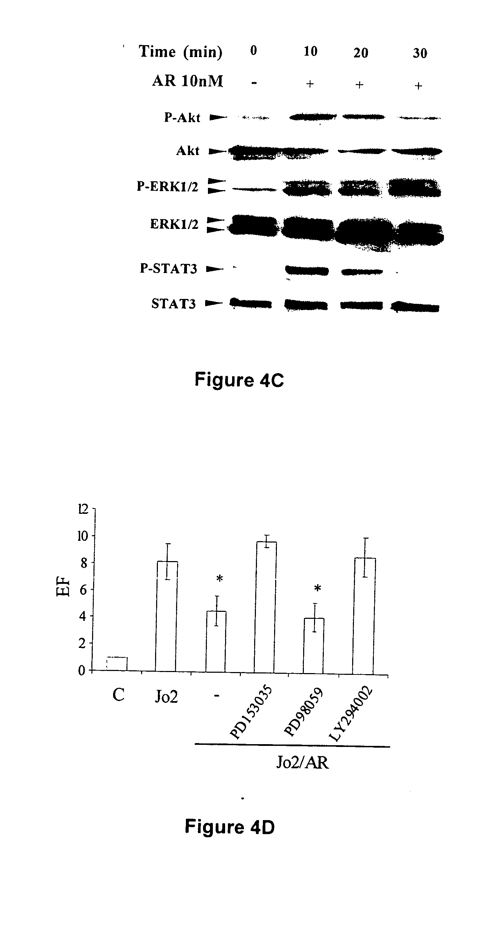

**Figure 4C:** Activation of antiapoptotic signaling pathways by AR in cultured mouse hepatocytes. Akt, ERK1/2 and STAT3 phosphorylation state was evaluated via Western blotting in extracts of mouse hepatocytes at different times after addition of AR (20 nM). Representative images are shown of three experiments performed in duplicate.
**Figure 4D:** Effect of AR (20 nM) on apoptosis induced by actinomycin D and Jo-2 in mouse hepatocytes cultured in the presence of EGF-R inhibitor PD153035 (1 µM), MEK1 inhibitor PD98059 (10 µM) or PI-3K inhibitor LY-294002 (20 µM). Values are expressed as the mean ± SEM of three experiments performed in triplicate, and the asterisk indicates P<0.05 vs Jo2.

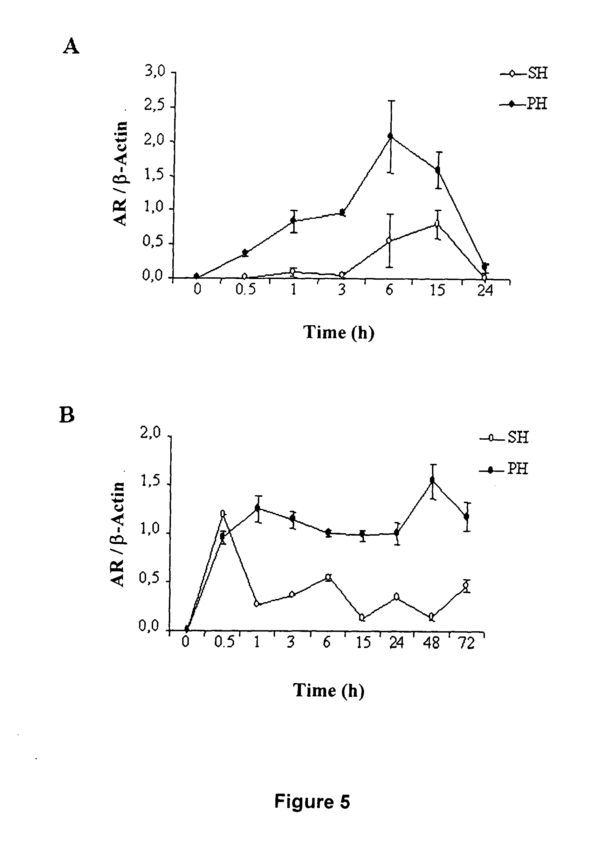

**Figures 5A, 5B:** AR gene expression in rat liver (Fig. 5 A) and mouse liver (Fig. 5B) after partial hepatectomy (PH). The levels of mRNA encoding for AR were analyzed at different times after PH in the remaining liver parenchyma via PCR in real time. The values are expressed as the mean ± SEM of three different animals. The closed circles correspond to hepatectomized animals, the open circles correspond to animals subjected to sham operations.

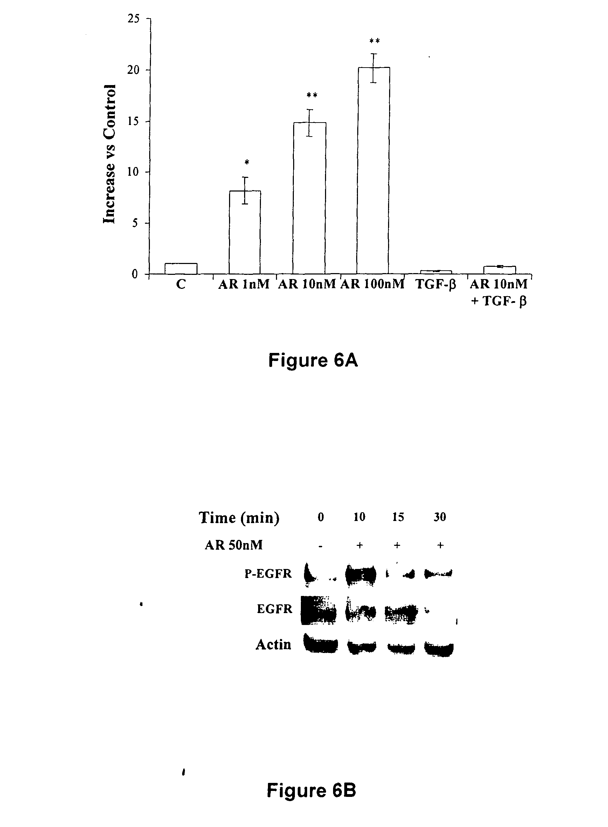

**Figure 6A:** Stimulation of DNA synthesis by AR in cultured rat hepatocytes. Hepatocytes were treated with increasing concentrations of AR, and DNA synthesis was measured by determining the incorporation of [³H]thymidine. The effect of TGFβ (8 ng/ml) on DNA synthesis induced by AR is shown. Values are expressed as the mean ± SEM of three experiments performed in quadruplicate. One asterisk indicates P<0.05 and two asterisks indicate P<0.01 *vs* control.
**Figure 6B:** Stimulation of EGF-R phosphorylation on tyrosine residues by AR in cultured rat hepatocytes. Tyrosine-phosphorylated EGF-R and total EGF-R were detected by Western blotting. Representative images are shown of three experiments performed in duplicate.

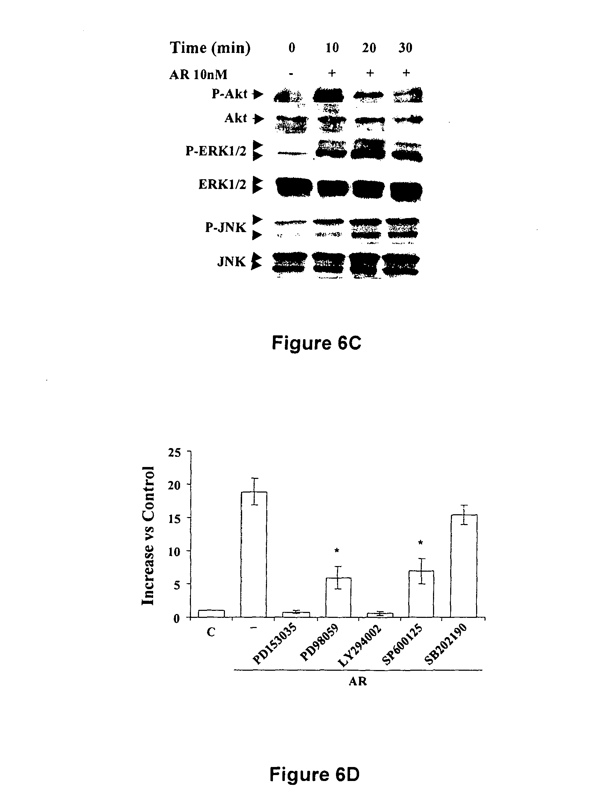

**Figure 6C:** Activation of signaling pathways related to cell proliferation by AR in cultured rat hepatocytes. Akt, ERK1/2 and JNK phosphorylation state was evaluated via Western blotting using specific antibodies in rat hepatocyte extracts at different times after addition of AR. Representative images are shown of three experiments performed in duplicate.
**Figure 6D:** Effect of AR (100 nM) on DNA synthesis in rat hepatocytes in the presence of EGF-R inhibitor PD153035 (1 µM), MEK1 inhibitor PD98059 (10 µM), PI-3K inhibitor LY294002 (20 µM), JNK inhibitor SP600125 (20 µM), or p38 MAPK inhibitor SB202190 (25 µM). The asterisks indicate P<0.05 vs AR alone. Values are expressed as the mean ± SEM of three experiments performed in triplicate.

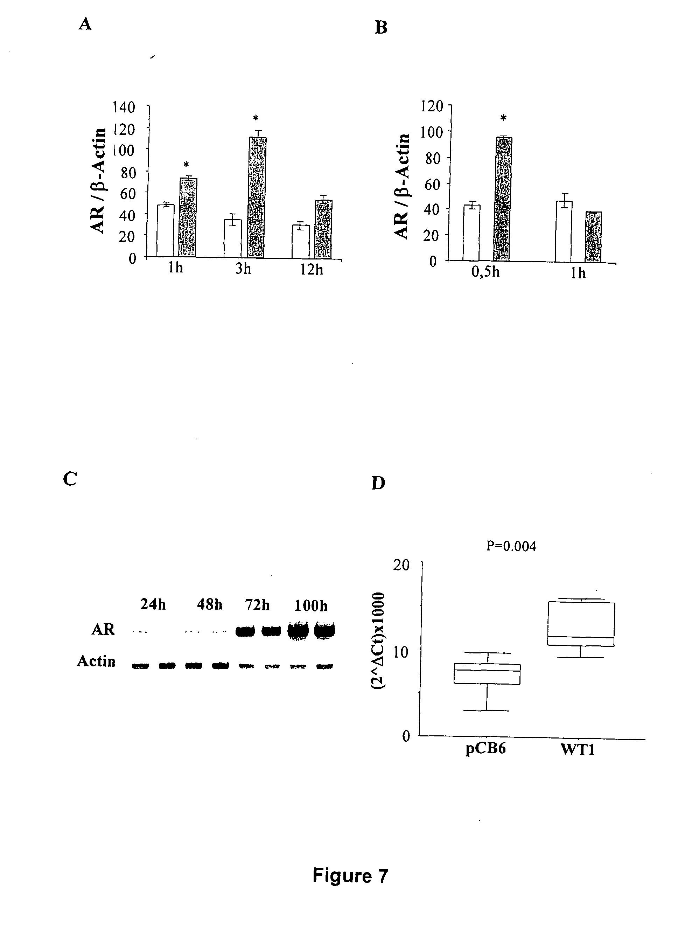

**Figure 7A:** AR gene expression in cultured rat hepatocytes treated with IL-1β (2 ng/ml) (closed bars) during different periods of time. Genic expression of AR was determined by real time PCR. The asterisk indicates P<0.05 vs controls (open bars). Values are expressed as the mean ± SEM of three experiments performed in triplicate.
**Figure 7B:** AR gene expression in cultured rat hepatocytes treated with PGE₂ (10 µM) (closed bars) during different periods of time. The gene expression of AR was determined by PCR in real time. The asterisk indicates P<0.05 vs controls (open bars). Values are expressed as the mean ± SEM of three experiments performed in triplicate.
**Figure 7C:** Analysis of the baseline gene expression of AR in rat hepatocytes as a function of the duration of culture, via RT-PCR. A representative experiment is shown.
**Figure 7D:** AR gene expression in cultured rat hepatocytes 24 h after transfection with an equimolar mixture of pCB6 plasmids encoding for the four isoforms of WT1, or with an equivalent amount of the gutless vector pCB6. AR gene expression was determined by real time PCR.

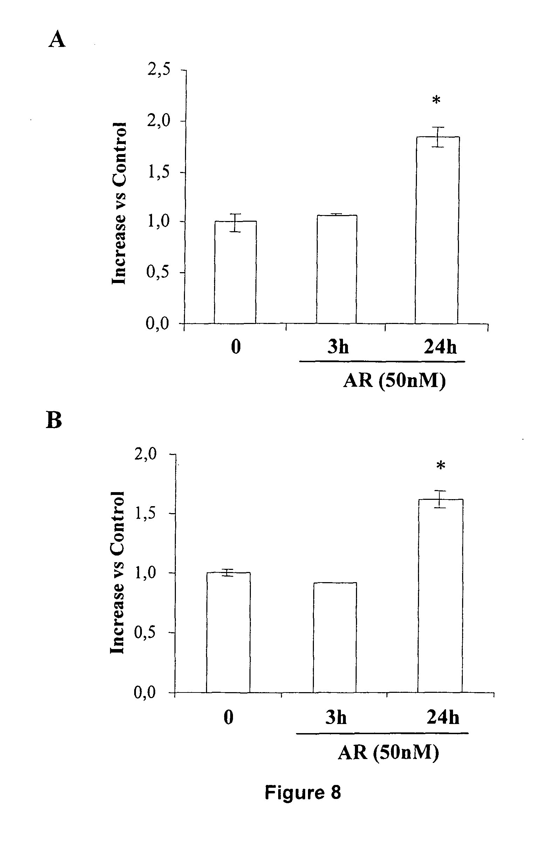

**Figures 8A, 8B:** TGFα (Figure 8A) and CT-1 (Figure 8B) gene expression in cultured rat hepatocytes treated with AR during different periods of time. Values are expressed as the mean ± SEM of three experiments performed in triplicate, and are expressed as the number-fold increment *vs* controls corresponding to each time point. The asterisk indicates P<0.05 vs controls.

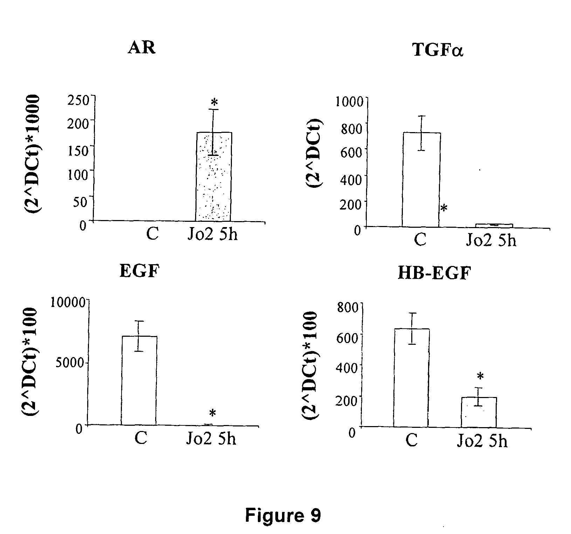

**Figure 9:** Gene expression of EGF-R, AR, TGFα, EGF and HB-EGF ligands in the liver of control mice -C- and in the liver of mice treated with Jo2 antibody. The expression of these genes was determined by RT-PCR. The asterisk indicates *P*<0.01 *vs* controls. Values are expressed as the mean ± SEM of three experiments performed in triplicate.

### TRIALS

AR gene expression is detected in cirrhotic human liver, and is rapidly induced in experimental liver damage and following partial hepatectomy. The experiments carried out by the inventors, together with an exhaustive analysis of the results obtained which constitute the scientific basis and support for the use of AR as contemplated in the present invention, are presented below.

### AR gene expression induced in chronic and acute liver damage

The inventors had already shown expression of transcription factor *WT1* to be induced in almost all tested samples of cirrhotic human liver and in cirrhosis induced by CCl₄ in rats (8). The fact that AR is a principal transcription target for *WT1* (10) led the inventors to examine the expression of this growth factor in the liver of cirrhotic patients. Although real time PCR analysis (RTi-PCR) yielded barely detectable levels of AR expression in healthy human liver, high levels of mRNA encoding for AR were observed in approximately 75% of the patients with cirrhosis (Figure 1A). Of great interest is the fact that levels of AR gene expression were directly correlated to those of WT1 gene in control livers and in cirrhotic individuals (*r*=0.752, P<0.001). Coinciding with the data in humans, AR expression also increased in experimental cirrhosis induced in rats following administration of CCl₄ (Figure 1B) and bile duct ligation (not shown).
In order to determine whether AR could form part of the rapid liver response to aggression, evaluations were made of the levels of mRNA encoding AR, and of protein in the mouse liver after administration of a single intraperitoneal injection of CCl₄ (1 µl/g), or after injection of Jo2 antibody (4 µg/mouse). Figure 2A shows that levels of mRNA encoding AR were intensely induced 5 h after the start of treatments. Western blot was performed with samples of liver obtained 12 and 24 h after administration of antibody Jo2 or CCl₄, respectively. Using an affinity purified and biotinylated anti-AR antibody, a set of proteins was detected that were only present in the liver of treated mice (Figure 2B). The four bands of approximately 50, 43, 28 and 19 kDa are consistent with the different forms of AR described in epithelial cells [(22) Brown, C.L., Meise, K.S., Plowman, G .D., Coffey, R.J., Dempsey, P.J. 1998. Cell surface ectodomain cleavage of human amphiregulin precursor is sensitive to a metalloprotease inhibitor. J. Biol. Chem. 273: 17258-17268]. The bands corresponding to 50 and 28 kDa probably represent forms of AR anchored to the membrane, while the bands corresponding to 43 and 19 kDa can be soluble forms of AR processed proteolytically (22).

### Attenuation of acute liver damage induced by CCl₄ or activation of Fas in mice via the administration of AR

In order to determine whether AR can limit the extent of liver damage, the inventors have examined the effect of the administration of AR in mice subjected to acute liver damage secondary to treatment with CCl₄ or antibody Jo2. CCl₄ induces liver necrosis and apoptosis due to cellular, lysosomal and mitochondrial membrane permeability alterations [(23) Berger, M.L., Bhatt, H., Combes, B., Estabrook, R. 1986. CCl4-induced toxicity in isolated hepatocytes: the importance of direct solvent injury. Hepatology 6: 36-45. (24) Kovalovich, K., Li, W., DeAngelis, R., Greenbaum, L.E., Ciliberto, G., and Taub, R. 2001. Interleukin-6 protects against Fas-mediated death by establishing a critical level of anti-apoptotic hepatic proteins FLIP, Bcl-2, and Bcl-xL. J. Biol. Chem. 276: 26605-26613. (25) Shi, J., Aisaki, K., Ikawa, Y., Wake, K. 1998. Evidence of hepatocyte apoptosis in rat liver after the administration of carbon tetrachloride. Am. J. Pathol. 153: 515-525. (26) Kovalovich, K., DeAngelis, R.A., Li, W., Furth, E.E., Ciliberto, G., Taub, R. 2000. Increased toxin-induced liver injury and fibrosis in interleukin-6-deficient mice. Hepatology 31: 149-159. (27) Czaja, M.J., Xu, J., Alt, E. 1995. Prevention of carbon tetrachloride-induced rat liver injury by soluble tumor necrosis factor receptor. Gastroenterology 108: 1849-1854]. The serum levels of both AST and ALT increased appreciably 24 h after injection of CCl₄. This increase, however, was clearly attenuated in mice treated with AR (Figure 3A). Consistent with this, the degree of histological damage decreased in the mice treated with AR (Figure 3A).

Serum AST and ALT levels increased considerably 12 h after injection of Jo2 (Figure 3B). Treatment with AR strongly suppressed serum transaminase elevation, and histopathological analysis confirmed that administration of AR almost completely avoided liver damage (Figure 3B). Apoptotic cell death is a principal determinant in liver damage mediated by Fas [(5)-(7), (28) Ogasawara, J., Watanabe-Fukunaga, R., Adachi, M., Matsuzawa, A., Kasugai, T., Kitamura, Y., Itoh, N., Suda, T., Nagata, S.1993. Lethal effect of the anti-Fas antibody in mice. Nature 364: 806-809. (29) Nagata, S. 1997. Apoptosis by death factor. Cell 88: 355-365]. In order to confirm that the hepatoprotective effects of AR against liver damage mediated by Fas originate from antiapoptotic activity, the inventors measured the proteolytic activation of caspase-3 and its activity in mouse liver extracts. They found that induction of caspase-3 activity detected in mice treated with antibody Jo2 was strongly inhibited by administration of AR (Figure 3C). Using an antibody that specifically recognises the p17 subunit of active caspase-3, the rupture of caspase-3 was seen to be avoided by treatment with AR - thus suggesting specific AR-mediated block of the apoptotic route induced by Fas (Figure 3D). Proteins of the Bcl-2 family inhibit apoptosis induced by a variety of stimuli, including apoptosis mediated by Fas [(30) Shimizu, S., Eguchi, Y., Kosaka, H., Kamiike, W., Matsuda, H., Tsujimoto, Y. 1995. Prevention of hypoxia-induced cell death by Bcl-2 and Bcl-xL. Nature 374: 811-813. (31) Stoll, S.W., Benedict, M., Mitra, R., Hiniker, A., Elder, J.T., Nuñez, G. 1998. EGF receptor signaling inhibits keratinocyte apoptosis: evidence for mediation by Bcl-xL. Oncogene 16: 1493-1499. (32) Lacronique, V., Mignon, A., Fabre, M., Viollet, B., Rouquet, N., Molina, T., Porteu, A., Henrion, A., Bouscary, D., Varlet, P., Joulin, V., Kahn, A. 1996. Bcl-2 protects from lethal hepatic apoptosis induced by an anti-Fas antibody in mice. Nat. Med. 2: 80-86]. The expression of Bcl-x_{L} protein were evaluated via Western blot 6 h after injection of Jo2 antibody. The levels of Bcl-x_{L} protein were greater in the liver of mice treated with AR and antibody Jo2 versus mice treated with antibody Jo2 alone (Figure 3D).

### Direct antiapoptotic effect of AR on hepatocytes in primary culture

In order to determine whether the *in vivo* antiapoptotic effects of AR could be mediated by direct action of AR on liver parenchymal cells, the inventors used mouse hepatocytes in primary culture. It has been reported that hepatocytes exposed to Jo-2 antibodies undergo apoptosis effectively in presence of actinomycin D [(5), (6), (33) Ni, R., Tomita, Y., Matsuda, K., Ichiara, A., Ishimura, K., Ogasawara, J., Nagata, S. 1994. Fas-mediated apoptosis in primary cultured mouse hepatocytes. Exp. Cell Res. 215: 332-337]. Hepatocytes were pretreated with different concentrations of AR during 3 h before addition of actinomycin D and Jo2 antibody. Measurements of apoptosis and related molecular events, were made 18 h later. As can be seen in Figure 4A, hepatocytes were protected from apoptosis by AR in a dose-dependent manner, thus indicating a direct cytoprotective effect of AR in the prevention of Fas-mediated liver cell apoptosis. AR-mediated cytoprotective activity was also seen in apoptosis induced by other agents such as TNFα plus galactosamine, okadaic acid and transforming growth factor β (TGFβ)(data not shown). Based on the antiapoptotic effect of AR, the inventors found the proteolysis and activation of caspase-3 induced by Jo2 antibody to be significantly inhibited by AR (Figure 4b). Likewise, they found antiapoptotic protein Bcl-x_{L} to be induced by treatment with AR in mouse hepatocytes treated with Jo2 (Figure 4B). In order to identify the AR antiapoptotic signaling mechanisms, PI-3K/Akt and ERK1/2 pathways were studied, as they are general mediators of cell survival [(4), (6)]. The cultured mouse hepatocytes treated with AR showed an increased phosphorylation of Akt and ERK1/2 (Figure 4 C). A key signaling molecule implicated in protection against liver damage induced by Fas is STAT3 [(34) Shen, Y., Devgan, G., Darnell, J.E., Bromberg, J.F. 2001. Constitutively activated Stat3 protects fibroblasts from serum withdrawal and UV-induced apoptosis and antagonizes the proapoptotic effects of activated Stat1. Proc. Natl. Acad. Sci. USA. 98: 1543-1548. (35) Haga, S., Terui, K., Zhang, H.Q., Enosawa, S., Ogawa, W., Inoue, H., Okuyama, T., Takeda, K., Akira, S., Ogino, T., et al. 2003. Stat3 protects against Fas-induced liver injury by redox-dependent and -independent mechanisms. J. Clin. Invest. 112: 989-998]. AR was seen to stimulate phosphorylation of STAT3 (Figure 4C).

EGF-R activation by AR seems to be essential in mediating the antiapoptotic effect of this growth factor on cell death induced by Fas. This became evident when mouse hepatocytes were pretreated during 1 h with EGF-R inhibitor PD153035, before adding AR, and the protection afforded by AR was lost (Figure 4D). In order to avoid apoptosis it also proved necessary for AR to activate the PI-3K/Akt route, which operates below EGF-R. This was shown by the marked inhibitory effect of PI-3K inhibitor LY294002 on the protection afforded by AR (Figure 4D). However, MEK1 inhibitor PD98059 did not interfere with the antiapoptotic effect of AR (Figure 4D).

### AR expression in the liver after partial hepatectomy

The present inventors also examined AR expression in mouse and rat liver after two-thirds partial hepatectomy [(1), (4)]. As can be seen in Figure 5A, AR mRNA was not detectable in rat liver before PH, though its appearance was detected half an hour after the operation - reaching peak value after 6 hours followed by a gradual decline in expression between 15 and 24 h. Interestingly, expression of AR gene in rats subjected to sham operations (SH) was transiently induced after between 6 and 15 h (Figure 5A). The expression of the AR gene in mouse liver was also soon induced after PH. Levels of mRNA encoding AR were seen to increase 0.5 h after PH, reaching a peak at between 24 and 48 h, and decreasing thereafter (Figure 5B). Expression of AR gene was also induced in mice subjected to sham operations, though the kinetics were much faster than in rats. At the shortest timepoint evaluated (0.5 h), mice subjected to sham operations showed levels of mRNA encoding AR similar to those of hepatectomised animals. However, 1 h after the intervention, the levels of mRNA encoding AR decreased significantly in mice subjected to sham operations in comparison with animals subjected to resection, and this situation persisted throughout the rest of the study (Figure 5B).

### AR mediated induction of DNA synthesis in isolated hepatocytes

Once the inventors had shown that expression of AR gene is rapidly induced in liver damage and PH, and that AR can play a protective role for the liver parenchyma, they attempted to demonstrate that AR can also have mitogenic behavior for hepatocytes.

As can be seen in Figure 6A, AR behaves as a pure mitogen for isolated hepatocytes in primary culture, stimulating the incorporation of [³H]thymidine to DNA in a dose-dependent manner. The effect of AR upon DNA synthesis was abrogated by TGFβ, a growth factor implicated in physiological termination of liver regenerative response (1).

AR is an EGF-R ligand, a receptor abundantly expressed by hepatocytes in the adult animal [(36) Salomon, D.S., Brandt, R., Ciardiello, F., Normanno, N. 1995. Epidermal growth factor-related peptides and their receptors in human malignancies. Crit. Rev. Oncol. Hematol. 19: 183-232. (37) Carver, R.S., Stevenson, M.C., Scheving, L.A., and Russell, W.E. 2002 Diverse expression of ErbB receptor proteins during rat liver development and regeneration. Gastroenterology 123: 2017-2027]. The inventors examined the intracellular signaling of AR in cultured rat hepatocytes. Treatment of isolated rat hepatocytes with AR induced rapid and transient phosphorylation of EGF-R (Figure 6B). An analysis was made of the pathways of mitogen-activated protein kinase (MAPK) and phosphatidylinositol 3-kinase (PI-3K), as the main signaling cascades in the mitogenic response of hepatocytes to growth factors [(38) Band, C.J., Mounier, C., Posner, B. 1999. Epidermal growth factor and insulin-induced deoxyribonucleic acid synthesis in primary rat hepatocytes is phosphatidylinositol 3-kinase dependent and dissociated from protooncogene induction. Endocrinology 140: 5625-5634. (39) Coutant, A., Rescan, C., Gilot, D., Loyer, P., Guguen-Guillouzo, C., Baffet, G. 2002. PI3K-FRAP/mTOR pathway is critical for hepatocyte proliferation whereas MEK/ERK supports both proliferation and survival. Hepatology 36: 1079-1088]. More recently, it has been shown that activation of c-Jun-N-terminal kinase (JNK) significantly contributes to hepatocyte proliferation after PH [(40) Schwabe, R.F., Bradham, C.A., Uehara, T., Hatano, E., Bennett, B.L., Schoonhoven, R., Brenner, D.A. 2003. c-Jun-N-Terminal kinase drives cyclin D1 expression and proliferation during liver regeneration. Hepatology 37: 824-832]. As observed in mouse hepatocytes, the treatment of isolated rat hepatocytes with AR rapidly induces phosphorylation of ERK1/2 and Akt (Figure 6C). In addition, the inventors have observed JNK phosphorylation in response to treatment with AR (Figure 6C).

To evaluate AR signaling in hepatocyte proliferation, the inventors measured [³H]thymidine incorporation in the DNA of rat hepatocytes treated with AR in the presence of inhibitors of these signaling pathways. As can be seen in Figure 6D, the the EGF-R tyrosine kinase activity inhibitor, PD153035, completely prevents DNA synthesis stimulated by AR. A similar degree of inhibition was observed for PI-3K inhibitor, LY294002, while MEK1 inhibitor, PD98059, and JNK inhibitor, SP600125, reduced the effects of AR by 70% (Figure 6D). However, treatment with p38-MAPK inhibitor, SB202190, exerted no significant effect on DNA synthesis stimulated by AR (Figure 6D).

### AR gene expression in isolated hepatocytes

The inventors have shown that AR is expressed in the liver under different situations of injury and regeneration of liver tissue. In order to identify the mechanisms responsible for AR induction, rat liver parenchymal cells were isolated, and AR gene expression was examined under different conditions. Firstly, the effect of different factors implicated in liver inflammatory and regenerative processes, such as IL-1β, IL-6, TNFα, HGF and prostaglandin E2 (PGE₂) was evaluated [(1)-(4), (41) Rudnick, D.A., Perlmutter, D.H., and Muglia, L.J. 2001. Prostaglandins are required for CREB activation and cellular proliferation during liver regeneration. Proc. Natl. Acad. Sci. USA. 98: 8885-8890]. Among the cytokines and growth factors evaluated, IL-1β, was the only molecule found to stimulate AR gene expression (Figure 7A). Based on earlier observations in colon cancer cells [(42) Shao, J., Lee, S.B., Guo, H., Evers, M., and Sheng, H. 2003. Prostaglandin E2 stimulates the growth of colon cancer cells via induction of amphiregulin. Cancer Res. 63: 5218-5223], the treatment of rat hepatocytes with PGE₂ was seen to give rise to a rapid induction of AR gene expression (Figure 7B). It has been postulated that PGE₂ mediated stimulation of AR gene expression is induced by the cAMP/protein kinase A (PKA) pathway, which acts upon a cAMP response element (CRE) in the AR promoter (42). In consistence with this mechanism, the inventors found that the cAMP inducer, forskolin, promoted expression of the AR gene in isolated hepatocytes (data not shown). The treatment of hepatocytes with different oxidative stress inducers, such as hydrogen peroxide and menadione, exerted no effect upon expression of the AR gene (data not shown).

It was also seen that expression of AR gene was induced in cultured hepatocytes, and that the magnitude of this effect increased with time in culture (Figure 7C). AR is a *bona* fide target for WT1 transcription factor (10). The inventors have found that transfection of hepatocytes with an equimolar mixture of plasmids encoding the four isoforms of WT1 gave rise to an increase in the levels of mRNA encoding AR determined by real time PCR. Although these data show that WT1 can control expression of AR gene in isolated hepatocytes, the induction of AR in cultured hepatocyte precedes that of WT1 (data not shown) - thus indicating that the previously identified factors, IL-1β and PGE2, could be responsible for the rapid initial induction of AR gene expression in cultured hepatocytes.

### AR induction of the expression of liver protection and regeneration mediators in isolated hepatocytes

In order to explore the mechanisms underlying the hepatoprotective effects of AR in more depth, the inventors evaluated the effects of this growth factor on the expression of TGFα and cardiotrophin-1 (CT-1), key molecules implicated in the endogenous response to liver damage and PH [(7),(93) Bustos, M., Beraza, N., Lasarte, J-J., Baixeras, E., Alzuguren, P., Bordet, T., Prieto, J. 2003. Protection against liver damage by cardiotrophin-1: a hepatocyte survival factor up-regulated in the regenerating liver in rats. Gastroenterology 125: 192-201. (44) Webber, E.M., Fitzgerald, M.J., Brown, P.I., Bartlett, M.H., Fausto, N. 1993. Transforming growth factor-α expression during liver regeneration after partial hepatectomy and toxic injury, and potential interactions between transforming growth factor-α and hepatocyte growth factor. Hepatology 18: 1422-1431]. AR treatment of isolated rat hepatocytes increased the levels of mRNA encoding TGFα and CT-1 (Figure 8A and B). These responses emphasize the relevance of AR as a hepatotrophic factor.

### Expression of EGF-R ligands in acute liver damage mediated by Fas

In addition to AR, EGF-R can be activated by a family of ligands that together with EGF and TGFα include heparin binding EGF type growth factor (HB-EGF)[(36), (45) Holbro, T., Hynes, N.E. 2004. ErbB receptors: directing key signaling networks throughout life. Annu. Rev. Pharmacol. Toxicol. 44: 195-217]. For a more in-depth evaluation of the relative contribution of these EGF-R ligands to the rapid hepatoprotective and regenerative response that follows acute liver damage, the inventors examined their gene expression profiles in mice treated with Jo2 antibodies. As is shown in Figure 9, EGF, TGFα and HB-EGF expression was detected via real time PCR in the liver of control mice, which previously presented undetectable levels of expression of mRNA encoding AR. Five hours after administration of Jo2 antibody, mRNA levels corresponding to EGF, TGFα and HB-EGF decreased significantly, while appreciable induction of AR gene expression was observed. These findings globally indicate that AR is the only examined EGF-R ligand induced in mouse liver during the early phases of acute liver damage.

As a summary of the above, it has been shown that AR gene expression is rapidly and consistently induced in different liver damage models, and that exogenous administration of AR affords significant liver protection.

The above observations globally indicate in a clear and conclusive manner that AR can be regarded as a new active participant in the complex process of liver regeneration. Accordingly, AR appears to offer enormous therapeutic potential for the management of pathological situations produced by acute liver damage - with particular emphasis on critical situations such as ALF.

### EMBODIMENTS OF THE INVENTION

The present invention is additionally illustrated by the following example, which in combination with the above-described figures, shows the experimental methodology used to develop the present invention. It is understood that experts in the field will comprehend the modifications and changes that may be made within the scope of the present invention.

### EXAMPLE

### Patients

Samples of liver tissue were obtained from two groups of subjects: (a) controls (n=26; 19 males; mean age 50.8 years, range 18-73 years) with minimal liver alterations. Tissue samples were obtained as a result of digestive tract tumor surgery (16 cases) or from percutaneous liver biopsies performed due to small alterations in liver function test parameters (10 cases); and (b) liver cirrhosis (n=29; 24 males; mean age 56, range 36-77 years) attributable to hepatitis C virus (HCV) infection in 8 cases, alcoholism in 13 cases, hepatitis B virus (HBV) infection in 3 cases, autoimmune hepatitis in 3 cases, hemochromatosis in one case and cryptogenic hepatitis in another case. Associated hepatocellular carcinoma (HCC) was present in 10 cirrhotic patients. This study was approved by the Human Research Review Committee of the University of Navarra, Spain, and complied with the principles of the Declaration of Helsinki.

### Animal models

The experiments were carried out in compliance with the guidelines of the University of Navarra relating to the use of laboratory animals. Cirrhosis was induced with CCl₄ in male Wistar rats as described elsewhere [(16) Castilla-Cortazar, I., Garcia, M., Muguerza, B., Quiroga, J., Perez, R., Santidrian, S., Prieto, J. 1997. Hepatoprotective effects of insulin-like growth factor I in rats with carbon tetrachloride-induced cirrhosis. Gastroenterology 113:1682-1691]. Two-thirds PH or sham operations were performed on male Wistar rats (150 g) and male C57/BL6 mice (20 g) according to the method of Higgins and Andersen [(17) Higgins, G. M., Andersen, R. M. 1931. Experimental pathology of liver: restoration of liver of the white rat following partial surgical removal. Arch. Pathol. 12:186-202. (18) Latasa, M.U., Boukaba, A., Garcia-Trevijano, E.R., Torres, L., Rodriguez, J.L., Caballeria, J., Lu, S.C., López-Rodas, G., Franco, L., Mato, J.M., et al. 2001. Hepatocyte growth factor induces MAT2A expression and histone acetylation in rat hepatocytes. Role in liver regeneration. FASEB J. 10.1096/fj.00-0556fje. (19) Chen, L., Zeng, Y., Yang, H., Lee, T.D., French, S.W., Corrales, F.J., Garcia-Trevijano, E.R., Avila, M.A., Mato, J.M., and Lu, S.C. 2004. Impaired liver regeneration in mice lacking methionine adenosyltransferase 1A. FASEB J. 18: 914-916] . Following sedation, in the sham operated animals the liver was exposed and then returned to the abdominal cavity. Acute liver damage was induced in male C57/BL6 mice (20 g)(n= 3-5 per condition and time point) by means of a single intraperitoneal injection of CCl₄ (1 µl/g body weight in olive oil) (Sigma, St. Louis, MO, USA) or Jo2 monoclonal antibody (4 µg/mouse in saline solution)(BD PharMingen, San Diego, CA, USA) [(5),(20) Martinez-Chantar, M.L., Corrales, F.J., Martinez-Cruz, A., Garcia-Trevijano, E.R., Huang, Z.Z., Chen, L.X., Kanel, G., Avila, M.A., Mato, J.M., Lu, S.C. 2002. Spontaneous oxidative stress and liver tumors in mice lacking methionine adenosyltransferase 1A. FASEB J. 10.1096/fj.02-0078fje]. The controls received an equivalent volume of olive oil or saline solution. In the cases indicated, the mice received an intraperitoneal injection of human recombinant AR (9.5 µg/mouse) (Sigma) 6 and 0.5 h before and 3 h after Jo2 antibody, or 0.5 h before and 12 h after CCl₄. At the indicated timepoints, mice were subjected to blood sampling and the sera were analysed for alanine and aspartate aminotransferase (ALT and AST) as described elsewhere [(16) and (21) Lasarte, J.J., Sarobe, P., Boya, P., Casares, N., Arribillaga, L., López-Díaz of Cerio, A., Gorraiz, M., Borrás-Cuesta, F., Prieto, J. 2003. A recombinant adenovirus encoding hepatitis C virus core and E1 proteins protects mice against cytokine-induced liver damage. Hepatology 37: 461-470]. Mice were sacrificed by cervical dislocation, and the livers were quickly frozen in liquid nitrogen, or fixed in formalin and embedded in paraffin for staining with hematoxylin and eosin (H&E).

### Isolation, culture and treatment of rat and mouse hepatocytes

Hepatocytes were isolated from male Wistar rats (150 g) and C57/BL6 mice (20 g) by perfusion with collagenase (Gibco-BRL, Paisley, UK) as described elsewhere [(18), (20)]. Cells (5 x 10⁵ cells per well) were plated onto 6-well plates coated with collagen (type I collagen, Collaborative Biomedical, Bedford, MA, USA). Cultures were maintained in MEM medium supplemented with 10% fetal calf serum (FCS), non-essential amino acids, glutamine 2 mM and antibiotics (all supplied by Gibco-BRL). After 2 h of incubation, the medium was removed and cells were again cultured in the same medium with 5% FCS. Where applicable, hepatocytes were treated with IL-1β or TNFα from Roche (Mannheim, Germany), HGF or forskolin from Calbiochem (San Diego, CA, USA), IL6 from RD Systems (Wiesbaden-Nordenstadt, Germany), or PGE₂ from Alexis QBiogene (Carlsbad, CA, USA).

Apoptosis was induced in cultured mouse hepatocytes by treatment with 0.5 µg/ml of Jo2 antibody and 0.05 µg/ml of actinomycin D as described elsewhere (5). Where applicable, the hepatocytes were treated with AR 6 h before the addition of Jo2 antibody and actinomycin D. Apoptosis was estimated by determining soluble histone-DNA complexes using the Cell Death Detection Assay (Roche). ELISA tests for determining cell death were carried out following the manufacturer's instructions. The specific enrichment of mono- and oligonucleosomes released in the cytoplasm (enrichment factor, EF) was calculated as the ratio between the absorbance values of the samples corresponding to treated cells and control cells. An evaluation was also made of the effect of AR upon apoptosis mediated by Fas in the presence of MEK1 inhibitor, PD98059, PI-3K inhibitor, LY-294002, and the EGF-R tyrosine kinase activity inhibitor, PD153035 - all supplied by Calbiochem.

### Evaluation of DNA synthesis

For DNA synthesis, rat hepatocytes were plated to a density of 3 x 10⁴ cells/well in 96-well plates coated with collagen in MEM medium supplemented with 10% FCS. Five hours after plating, the medium was changed and cells were maintained in absence of serum for another 20 hours. DNA synthesis was assayed after 30 h of treatment with AR. A pulse of [³H]thymidine was administered (1 µCi/well)(Amersham Biosciences, Piscataway, NJ, USA) 22 h after the addition of AR. Cells were harvested, and the incorporation of thymidine was determined with a scintillation counter. Evaluations were made of the effect of AR on DNA synthesis in the presence of MEK1 inhibitor, PD98059, PI-3K inhibitor, LY294002, p38 MAPK inhibitor, SB202190, JNK inhibitor, SP600125, and of the EGF-R tyrosine kinase activity inhibitor, PD153035 - all supplied by Calbiochem.

### Transient transfection of rat hepatocytes

Rat hepatocytes in primary culture were transfected 24 h after isolation using Tfx^{™}-50 reagent (Promega, Madison, WI, USA) according to the manufacturer's instructions. The cells were transfected with an equimolar mixture of pCB6 plasmids encoding the four isoforms of *WT1* (characterised by the presence or absence of exons 5 and KTS), or with an equivalent amount of the gutless pCB6 vector, kindly provided by Dr. Jochemsen (Leiden University Medical Center, Leiden, The Netherlands). The efficacy of transfection of the equimolar mixture of the four isoforms of *WT1* was monitored by RT-PCR analysis using specific primers that discriminate between isoforms.

### RNA isolation and analysis of gene expression

Total RNA was extracted using TRI reagent (Sigma). Two µg of RNA were treated with DNase I (Gibco-BRL) before reverse transcription, for which M-MLV enzyme was used (Gibco-BRL) in the presence of RNase OUT (Gibco-BRL). For each PCR reaction 1/10 of each preparation of cDNA was used. PCR products were subjected to electrophoresis in 2% agarose gels, followed by staining with ethidium bromide and quantification using Molecular Analyst software (Bio-Rad, Hercules, CA, USA). Data were normalised with respect to β-actin gene expression levels. The study only included those samples with a mRNA amplification comparable amplification to β-actin. All primers were designed to distinguish between the amplification of genomic DNA and cDNA, and all products were sequenced to confirm specificity. The primers used are described below in Table I:

**Table I**

| **Primer** | **Sense (5'-3')** | **Antisense(5'-3')** |
|---|---|---|
| Human actin | AGCCTCGCCTTTGCCGA (SEQ ID NO:1) | CTGGTGCCTGGGGCG (SEQ ID NO:11) |
| Mouse actin | ACTGCGCTTCTTGCCGC (SEQ ID NO:2) | CATGACGCCCTGGTGTC (SEQ ID NO:12) |
| Rat actin | CAACCTCCTTGCAGCTC (SEQ ID NO:3) | CTGGTGCCTAGGGCG (SEQ ID NO:13) |
| Human AR | CATGCTGTGAGTTTTCATGGAC (SEQ ID NO:4) | CTGTCGCTCTTGATACTCGG (SEQ ID NO:14) |
| Rat and mouse AR | CTGCTGGTCTTAGGCTCAGG (SEQ ID NO:5) | CCAGGTTCTCGATGTATCTGC (SEQ ID NO:15) |
| CT1 | GTCTGGAAGACCACCAGACTG (SEQ ID NO:6) | AGCCGCTCGGACACCGGTAGC (SEQ ID NO:16) |
| EGF | CCCTGGATCCTATTACTGCAC (SEQ ID NO:7) | GAAAGCAATCACATTCCCAGG (SEQ ID NO:17) |
| HB-EGF | ATGAAGCTGCTGCCGTCGGTG (SEQ ID NO:8) | TGGATGCAGTAGTCCTTGTATT TC (SEQ ID NO:18) |
| TGFα | GCCCAGATTCCCACACTCAG (SEQ ID NO:9) | AGGACAGCCAGGGCCAC (SEQ ID NO:19) |
| WT1 | CGTTTCTCACTGGTCTCAGATG CCG (SEQ ID NO:10) | GGAATCAGATGAACTTAGGAG (SEQ ID NO:20) |

Real time PCR was performed using an iCycler (BioRad) and iQ SYBR Green Supermix (Bio-Rad). In order to monitor the specificity of final PCR products, the latter were analysed by fusion curves and electrophoresis. The amount of each transcript was expressed as n-fold the difference versus expression of the reference gene (β-actin)(2^{ΔCt}, where ΔCt represents the difference in threshold cycle between target genes and control gene).

### Measurement of caspase-3 activity

Caspase-3 activity in mouse hepatocytes and liver tissue lysates was assessed using the Caspase-3/CPP32 colorimetric assay kit (BioVision, Palo Alto, CA, USA). Cells in culture (5 x 10⁵ per condition) were lysed directly in the lysis buffer supplied with the kit after the corresponding treatments. Liver tissue was homogenised using a Dounce homogenizer in lysis buffer, followed by centrifugation at 15,000 rpm during 10 min. Cell lysates and supernatants from liver homogenates were used (200 µg in 50 µl) to measure caspase-3 activity following the manufacturer's instructions.

### Western blot

Homogenates from liver samples and isolated hepatocytes were subjected to Western blot analysis as described elsewhere [(19, (20)]. Antibodies used were: affinity purified and biotinylated polyclonal antibody specifically targeted to murine AR (BAF989)(RD Systems); specific antibodies for the p17 subunit of active caspase-3 (9664S), phosphorylated Akt (Ser⁴⁷³) (9271S) and phosphorylated STAT3 (Tyr⁷⁰⁵) (9131S) (Cell Signaling, Beverly, MA, USA); ERK1/2 (06-182), phosphorylated EGF-R (Tyr¹¹⁷³) (05-483) and STAT3 (06-596)(Upstate Biotechnology, Charlottesville, VA, USA). All other antibodies were from Santa Cruz Biotechnology (Santa Cruz, CA, USA): Bclx_{L} (sc8392), Bcl2 (sc7382), EGF-R (sc-03), phosphorylated ERK1/2 (Tyr²⁰⁴)(sc7383), Akt (sc5298), JNK (sc571), and phosphorylated JNK (Thr¹⁸³/Tyr¹⁸⁵) (sc6254).

### Statistical analysis

Data showing a normal distribution were compared between groups using an independent Student t-test and analysis of variance (ANOVA). Data without a normal distribution were compared using Kruskal-Wallis and Mann-Whitney tests. Correlations were evaluated by Spearman or Pearson correlation coefficients. Values of *P*<0.05 were considered significant. Data are expressed as the mean ± SEM, or as the median and interquartile range.

## Claims

1. Use of amphiregulin **characterised in that** amphiregulin is used in the preparation of a medicine for the treatment of acute liver damage.

2. Use of amphiregulin according to claim 1, **characterised in that** the medicine is useful for enhancing a primary endogenous protective reaction of liver tissue to acute liver damage.

3. Use of amphiregulin according to claim 1, **characterised in that** the medicine is useful for promoting DNA synthesis in hepatocytes.

4. Use of amphiregulin according to claim 1, **characterised in that** the medicine is useful for preventing hepatocyte death in liver tissue of patients with acute liver damage.

5. Use of amphiregulin according to claim 1, **characterised in that** the medicine is useful for stimulating regeneration of remaining liver parenchyma after acute liver damage of any etiology.

6. Use of amphiregulin according to claim 1, **characterised in that** the medicine is useful as a hepatoprotective drug for patients with acute liver damage of any etiology.

7. Use of amphiregulin according to claim 1, **characterised in that** the medicine is useful for stimulating liver regeneration after a partial hepatectomy.

8. Use of amphiregulin according to claim 1, **characterised in that** the medicine is useful as a hepatoprotective drug and as a stimulator of hepatocyte regeneration in patients receptors of a liver transplant *de vivo* or from a cadaver.


LIST OF SEQUENCES

<110> PROYECTO DE BIOMEDICINA CIMA, S.L.

<120> USE OF AMPHIREGULIN AS PROTECTIVE AGENT IN ACUTE LIVER DAMAGE

<130> Proyecto de Biomedicina CIMA

<140>
<141>

<160> 20

<170> PatentIn Ver. 2.1

<210> 1
<211> 17
<212> DNA
<213> Homo sapiens

<400> 1
agcctcgcct ttgccga
17

<210> 2
<211> 17
<212> DNA
<213> MOUSE

<400> 2
actgcgcttc ttgccgc
17

<210> 3
<211> 17
<212> DNA
<213> RAT

<400> 3
caacctcctt gcagctc
17

<210> 4
<211> 22
<212> DNA
<213> Homo sapiens

<400> 4
catgctgtga gttttcatgg ac
22

<210> 5
<211> 20

<212> DNA
<213> MOUSE OR RAT

<400> 5
ctgctggtct taggctcagg
20

<210> 6
<211> 21
<212> DNA
<213> Homo sapiens

<400> 6
gtctggaaga ccaccagact g
21

<210> 7
<211> 21
<212> DNA
<213> Homo sapiens

<400> 7
ccctggatcc tattactgca c
21

<210> 8
<211> 21
<212> DNA
<213> Homo sapiens

<400> 8
atgaagctgc tgccgtcggt g
21

<210> 9
<211> 20
<212> DNA
<213> Homo sapiens

<400> 9
gcccagattc ccacactcag
20

<210> 10
<211> 25
<212> DNA
<213> Homo sapiens

<400> 10
cgtttctcac tggtctcaga tgccg
25

<210> 11

<211> 15
<212> DNA
<213> Homo sapiens

<400> 11
ctggtgcctg gggcg
15

<210> 12
<211> 17
<212> DNA
<213> MOUSE

<400> 12
catgacgccc tggtgtc
17

<210> 13
<211> 15
<212> DNA
<213> RAT

<400> 13
ctggtgccta gggcg
15

<210> 14
<211> 20
<212> DNA
<213> Homo sapiens

<400> 14
ctgtcgctct tgatactcgg
20

<210> 15
<211> 21
<212> DNA
<213> MOUSE O RAT

<400> 15
ccaggttctc gatgtatctg c
21

<210> 16
<211> 21
<212> DNA
<213> Homo sapiens

<400> 16
agccgctcgg acaccggtag c
21

<210> 17
<211> 21
<212> DNA
<213> Homo sapiens

<400> 17
gaaagcaatc acattcccag g
21

<210> 18
<211> 24
<212> DNA
<213> Homo sapiens

<400> 18
tggatgcagt agtccttgta tttc
24

<210> 19
<211> 17
<212> DNA
<213> Homo sapiens

<400> 19
aggacagcca gggccac
17

<210> 20
<211> 21
<212> DNA
<213> Homo sapiens

<400> 20
ggaatcagat gaacttagga g
21